# EUROPEAN PATENT APPLICATION

(11) **EP 4 534 081 A1**
(43) Date of publication of application: **09.04.2025**
(21) Application number: 23835570.5
(22) Date of filing: 05.07.2023
(51) Int. Cl.: A61K 31/135, A61K 9/08, A61K 47/02, A61P 27/02

(54) **OPHTHALMIC COMPOSITION**

(30) Priority: 06.07.2022 JP 2022109256
(71) Applicant: Rohto Pharmaceutical Co., Ltd., Osaka-shi, Osaka 544-8666 (JP)
(72) Inventor: OGAWA Yoko, Osaka-shi, Osaka 544-8666 (JP); KITAZAWA Shota, Osaka-shi, Osaka 544-8666 (JP)
(74) Representative: Mewburn Ellis LLP
(86) International application number: PCT/JP2023/024965
(87) International publication number: WO 2024/010039

(57) **Abstract**

The present invention relates to an ophthalmic composition containing tramadol or a salt thereof, in which the ophthalmic composition has a pH of 4.0 to 6.8 and is stored in a container in which a portion in contact with the ophthalmic composition is partly or entirely formed of plastic.

## Description

### Technical Field

The present invention relates to an ophthalmic composition.

### Background Art

Tramadol is a non-narcotic analgesic classified as a weak opioid, and is used as a systemic analgesic for cancer pain (Non Patent Literature 1).

### Citation List

### Non Patent Literature

Non Patent Literature 1: Tramal (registered trademark) injection 100 medication package insert

### Summary of Invention

### Technical Problem

On the other hand, when tramadol is used as a therapeutic agent for a disease in an ophthalmic region, an ophthalmic preparation containing tramadol is required to have certain stability, but there has been no report so far on knowledge about the stability of the ophthalmic preparation containing tramadol. An object of the present invention is to provide a novel tramadol-containing ophthalmic composition having excellent stability.

### Solution to Problem

As a result of intensive studies to solve the above problems, the present inventors have found that when a pH of an ophthalmic composition containing tramadol is set to a specific range, the stability of the ophthalmic composition is significantly improved even when the ophthalmic composition is stored in a plastic container. The present invention is based on this finding and provides the following inventions.

[1] An ophthalmic composition comprising tramadol or a salt thereof, wherein the ophthalmic composition has a pH of 4.0 to 6.8 and is stored in a container in which a portion in contact with the ophthalmic composition is partly or entirely formed of plastic.
[2] The ophthalmic composition according to [1], wherein the plastic is at least one selected from the group consisting of polyethylene, polypropylene, and a cyclic olefin copolymer.
[3] The ophthalmic composition according to [1] or [2], further comprising a buffer.
[4] The ophthalmic composition according to any of [1] to [3], wherein a content of the tramadol or the salt thereof is 0.01 w/v% to 10 w/v% based on a total amount of the ophthalmic composition.
[5] A method for improving stability of tramadol or a salt thereof in an ophthalmic composition, the method comprising setting a pH of the ophthalmic composition comprising the tramadol or the salt thereof stored in a container formed of plastic to 4.0 to 6.8.
[6] The method according to [5], wherein the plastic is at least one selected from the group consisting of polyethylene, polypropylene, and a cyclic olefin copolymer.
[7] The method according to [5] or [6], wherein the ophthalmic composition further contains a buffer.

### Advantageous Effects of Invention

According to the present invention, a novel tramadol-containing ophthalmic composition having excellent stability can be provided.

### Description of Embodiments

Hereinafter, embodiments for carrying out the present invention will be described in detail. However, the present invention is not limited to the following embodiments.

In the present specification, a unit "%" of a content means "w/v%" and is synonymous with "g/100 mL" unless otherwise specified.

### [1. Ophthalmic Composition]

An ophthalmic composition according to the present embodiment contains (A) tramadol or a salt thereof (also referred to simply as "component (A)").

### [Component (A)]

Tramadol is a known compound represented by the following formula: 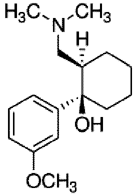 Note that, although the formula represents one of enantiomers for convenience, other enantiomers are also included in the present invention.

The salt of tramadol is not particularly limited as long as it is medicinally, pharmacologically (pharmaceutically), or physiologically acceptable. Specific examples of such a salt include a salt with an inorganic acid, a salt with an organic acid, a salt with an inorganic base, a salt with an organic base, a salt with an acidic amino acid, and a salt with a basic amino acid.

Examples of the salt with an inorganic acid include salts with hydrochloric acid, hydrobromic acid, sulfuric acid, nitric acid, phosphoric acid, and the like. Examples of the salt with an organic acid include salts with acetic acid, succinic acid, fumaric acid, maleic acid, tartaric acid, citric acid, lactic acid, stearic acid, benzoic acid, methanesulfonic acid (mesylic acid), ethanesulfonic acid, p-toluenesulfonic acid, and the like. Examples of the salt with an inorganic base include an alkali metal salt such as a sodium salt or a potassium salt, an alkaline earth metal salt such as a calcium salt or a magnesium salt, an aluminum salt, and an ammonium salt. Examples of the salt with an organic base include salts with diethylamine, diethanolamine, meglumine, N,N-dibenzylethylenediamine, and the like. Examples of the salt with an acidic amino acid include salts with aspartic acid, glutamic acid, and the like. Examples of the salt with a basic amino acid include salts with arginine, lysine, ornithine, and the like. As the salt of tramadol, a salt with an inorganic acid is preferable, and hydrochloride is more preferable.

The ophthalmic composition according to the present embodiment contains tramadol or a salt thereof as an active ingredient, and can be used for suppressing pain, for example.

A content of the component (A) in the ophthalmic composition according to the present embodiment is not particularly limited, and is appropriately set according to the type and content of other compounding ingredients, preparation form, and the like. The content of the component (A) may be 0.01 w/v% to 10 w/v%, 0.05 w/v% to 5 w/v%, 0.1 w/v% to 4 w/v%, or 3 w/v%, based on a total amount of the ophthalmic composition according to the present embodiment, from the viewpoint of more remarkably achieving the effect according to the present invention.

### [Buffer]

The ophthalmic composition according to the present embodiment preferably further contains a buffer. When the ophthalmic composition further contains a buffer, the effect of the present invention is more remarkably exhibited. The buffer is not particularly limited as long as it is medicinally, pharmacologically (pharmaceutically), or physiologically acceptable. Examples of the buffer include an inorganic buffer which is a buffer derived from an inorganic acid, and an organic buffer which is a buffer derived from an organic acid or an organic base.

Examples of the inorganic buffer include a boric acid buffer, a phosphoric acid buffer, and a carbonic acid buffer. Examples of the boric acid buffer include boric acid or a salt thereof (alkali metal borate, alkaline earth metal borate, or the like). Examples of the phosphoric acid buffer include phosphoric acid or a salt thereof (alkali metal phosphate, alkaline earth metal phosphate, or the like). Examples of the carbonic acid buffer include carbonic acid or a salt thereof (alkali metal carbonate, alkaline earth metal carbonate, or the like). In addition, as the boric acid buffer, the phosphoric acid buffer, or the carbonic acid buffer, a hydrate of boric acid, phosphoric acid, or carbonic acid may be used. As a more specific example, the boric acid buffer includes boric acid or a salt thereof (sodium borate, potassium tetraborate, potassium metaborate, ammonium borate, borax, or the like); the phosphoric acid buffer includes phosphoric acid or a salt thereof (disodium hydrogen phosphate, sodium dihydrogen phosphate, potassium dihydrogen phosphate, trisodium phosphate, tripotassium phosphate, calcium monohydrogen phosphate, calcium dihydrogen phosphate, or the like); and the carbonic acid buffer includes carbonic acid or a salt thereof (sodium bicarbonate, sodium carbonate, ammonium carbonate, potassium carbonate, calcium carbonate, potassium bicarbonate, magnesium carbonate, or the like).

Examples of the organic buffer include a citric acid buffer, an acetic acid buffer, a lactic acid buffer, a succinic acid buffer, a Tris buffer, and an AMPD buffer. Examples of the citric acid buffer include citric acid or a salt thereof (alkali metal citrate, alkaline earth metal citrate, or the like). Examples of the acetic acid buffer include acetic acid or a salt thereof (alkali metal acetate, alkaline earth metal acetate, or the like). Examples of the lactic acid buffer include lactic acid and a salt thereof (alkali metal lactate, alkaline earth metal lactate, or the like). Examples of the succinic acid buffer include succinic acid or a salt thereof (alkali metal succinate or the like). In addition, as the citric acid buffer, the acetic acid buffer, the lactic acid buffer, or the succinic acid buffer, a hydrate of citric acid, acetic acid, lactic acid, or succinic acid may be used. As a more specific example, the citric acid buffer includes citric acid or a salt thereof (sodium citrate, potassium citrate, calcium citrate, sodium dihydrogen citrate, disodium citrate, or the like); the acetic acid buffer includes acetic acid or a salt thereof (ammonium acetate, sodium acetate, potassium acetate, calcium acetate, or the like); the lactic acid buffer includes lactic acid or a salt thereof (sodium lactate, potassium lactate, calcium lactate, or the like); and the succinic acid buffer includes succinic acid or a salt thereof (monosodium succinate, disodium succinate, or the like). Examples of the Tris buffer include trometamol or a salt thereof (trometamol hydrochloride or the like). Examples of the AMPD buffer include 2-amino-2-methyl-1,3-propanediol or a salt thereof.

As the buffer, a boric acid buffer, a phosphoric acid buffer, and a citric acid buffer are preferable, a boric acid buffer and a phosphoric acid buffer are more preferable, and boric acid or a salt thereof and phosphoric acid or a salt thereof are still more preferable.

A commercially available buffer may be used. The buffers may be used alone or in combination of two or more types thereof.

A content of the buffer in the ophthalmic composition according to the present embodiment is not particularly limited, and is appropriately set according to the type of the buffer, the type and content of other compounding ingredients, the use and preparation form of the ophthalmic composition, and the like. The content of the buffer is, for example, preferably 0.01 w/v% to 10 w/v%, more preferably 0.05 w/v% to 5 w/v%, and still more preferably 0.1 w/v% to 3 w/v%, based on the total amount of the ophthalmic composition, from the viewpoint of more remarkably exhibiting the effect of the present invention. When the buffer is a boric acid buffer, the content is preferably 0.01 w/v% to 10 w/v%, more preferably 0.05 w/v% to 5 w/v%, still more preferably 0.1 w/v% to 3 w/v%, and particularly preferably 0.5 w/v% to 2.0 w/v%. When the buffer is a citric acid buffer or a phosphoric acid buffer, the content is preferably 0.01 w/v% to 10 w/v%, more preferably 0.05 w/v% to 5 w/v%, still more preferably 0.1 w/v% to 3 w/v%, still more preferably 0.1 w/v% to 1 w/v%, and particularly preferably 0.1 w/v% to 0.3 w/v%.

A content ratio of the buffer to the component (A) in the ophthalmic composition according to the present embodiment is not particularly limited, and is appropriately set according to the types of the component (A) and the buffer, the type and content of other compounding ingredients, the use and preparation form of the ophthalmic composition, and the like. From the viewpoint of further enhancing the effect of the present invention, the content ratio of the buffer to the component (A) may be, for example, 0.001 to 1,000 parts by mass, 0.01 to 100 parts by mass, or 0.025 to 30 parts by mass, with respect to 1 part by mass of the total content of the component (A) contained in the ophthalmic composition according to the present embodiment.

### [Inorganic Salt]

The ophthalmic composition according to the present embodiment may further contain an inorganic salt. When the ophthalmic composition further contains an inorganic salt, the effect of the present invention is more remarkably exhibited. The inorganic salt is not particularly limited as long as it is medicinally, pharmacologically (pharmaceutically), or physiologically acceptable.

Examples of the inorganic salt include chloride salts such as sodium chloride, potassium chloride, calcium chloride, and magnesium chloride. As the inorganic salt, sodium chloride and potassium chloride are preferable.

A commercially available inorganic salt may be used. The inorganic salts may be used alone or in combination of two or more types thereof.

A content of the inorganic salt in the ophthalmic composition according to the present embodiment is not particularly limited, and is appropriately set according to the type of the inorganic salt, the type and content of other compounding ingredients, the use and preparation form of the ophthalmic composition, and the like. The content of the inorganic salt is, for example, preferably 0.00001 w/v% to 3 w/v%, more preferably 0.0001 w/v% to 2 w/v%, and still more preferably 0.001 w/v% to 1.5 w/v%, based on the total amount of the ophthalmic composition, from the viewpoint of more remarkably exhibiting the effect of the present invention.

### [Preservative]

The ophthalmic composition according to the present embodiment may further contain a preservative. When the ophthalmic composition further contains a preservative, the effect of the present invention is more remarkably exhibited.

Examples of the preservative include a biguanide-based preservative such as chlorhexidine, alexidine, polyhexanide, or a salt thereof; a quaternary ammonium salt-based preservative such as benzalkonium chloride or benzethonium chloride; and a parabene-based preservative such as methyl parahydroxybenzoate, ethyl parahydroxybenzoate, propyl parahydroxybenzoate, or butyl parahydroxybenzoate.

As the preservative, a biguanide-based preservative is preferable, chlorhexidine or a salt thereof is more preferable, and chlorhexidine gluconate is still more preferable, from the viewpoint of more remarkably exhibiting the effect according to the present invention.

A commercially available preservative may be used. The preservatives may be used alone or in combination of two or more types thereof.

A content of the preservative in the ophthalmic composition according to the present embodiment is not particularly limited, and is appropriately set according to the type of the preservative, the type and content of other compounding ingredients, the use and preparation form of the ophthalmic composition, and the like. The content of the preservative is preferably 0.00001 w/v% to 2 w/v%, more preferably 0.00005 w/v% to 1 w/v%, and particularly preferably 0.00008 w/v% to 0.8 w/v%, based on the total amount of the ophthalmic composition, from the viewpoint of more remarkably exhibiting the effect of the present invention. As another aspect, 0.00005 w/v% to 0.5 w/v% and 0.0001 w/v% to 0.025 w/v% can also be presented as preferred contents.

A pH of the ophthalmic composition according to the present embodiment is 4.0 to 6.8. By setting the pH of the ophthalmic composition to be within the range, the stability of the ophthalmic composition containing tramadol or a salt thereof as an active ingredient is remarkably improved. From the viewpoint of further remarkably improving the stability of the ophthalmic composition, the pH of the ophthalmic composition is preferably 4.3 to 6.7, more preferably 4.5 to 6.6, still more preferably 5.0 to 6.6, still more preferably 5.0 to 6.5, particularly preferably 5.5 to 6.5, and most preferably 5.8 to 6.2. In addition, the pH of the ophthalmic composition according to the present embodiment may be 4.0 or more, 4.5 or more, 5.0 or more, 5.5 or more, 5.7 or more, or 5.8 or more, or may be 6.8 or less, 6.5 or less, 6.3 or less, 6.2 or less, or 6.0 or less.

The ophthalmic composition according to the present embodiment can be adjusted to an osmotic pressure ratio within a range acceptable to a living body as necessary. An appropriate osmotic pressure ratio can be appropriately set according to the use, preparation form, use method, and the like of the ophthalmic composition, and can be, for example, 0.4 to 5.0. The osmotic pressure ratio is defined as a ratio of an osmotic pressure of a sample to 286 mOsm (an osmotic pressure of a 0.9 w/v% aqueous sodium chloride solution) based on the Japanese Pharmacopoeia 18th Edition, and the osmotic pressure is measured with reference to the osmotic measurement method (freezing point depression method) described in the Japanese Pharmacopoeia. Note that, as the standard solution for measuring an osmotic pressure ratio (0.9 w/v% aqueous sodium chloride solution), sodium chloride (Japanese Pharmacopoeia standard reagent) is dried at 500 to 650°C for 40 to 50 minutes and then allowed to cool in a desiccator (silica gel), and 0.900 g of the dried solution is accurately weighed and dissolved in purified water to prepare exactly 100 mL of a solution, or a commercially available standard solution for measuring an osmotic pressure ratio (0.9 w/v% aqueous sodium chloride solution) can be used.

A viscosity of the ophthalmic composition according to the present embodiment is not particularly limited as long as it is within a medicinally, pharmacologically (pharmaceutically), or physiologically acceptable range. As the viscosity of the ophthalmic composition according to the present embodiment, for example, the viscosity at 20°C measured with a rotational viscometer (TV-20 type viscometer, manufactured by Toki Sangyo Co., Ltd., rotor; 1° 34' × R24) may be 1 to 10,000 mPa·s.

The ophthalmic composition according to the present embodiment can be prepared, for example, by adding and mixing the component (A) and, if necessary, other contained components so as to have a desired content. Specifically, for example, the ophthalmic composition can be prepared by dissolving or suspending the above components in purified water, and subjecting the solution to sterilization treatment by filtration sterilization or the like.

The ophthalmic composition according to the present embodiment can be in various dosage forms depending on the purpose, and examples thereof include a liquid preparation, a gel preparation, and a semi-solid preparation (ointment or the like).

When the ophthalmic composition according to the present embodiment is a liquid preparation, the ophthalmic composition for example, collyrium (also referred to as an eye drop or an eye lotion, and in addition, collyrium includes collyrium that can be applied while wearing contact lenses), an artificial tear solution, an eye wash (also called an eye wash solution or an eye wash drug, and in addition, the eye wash includes an eye wash that can wash the eye while wearing contact lenses). Note that the "contact lens" includes a hard contact lens and a soft contact lens (both ionic and nonionic lenses are encompassed, and both a silicone hydrogel contact lens and a non-silicone hydrogel contact lens are encompassed).

The ophthalmic composition according to the present embodiment is preferably collyrium (collyrium that can be instilled while wearing contact lenses) because the effect of the present invention can be more remarkably exhibited. When the ophthalmic composition according to the present embodiment is collyrium, the dosage and dose are not particularly limited as long as it is effective and has few side effects. For example, in the case of adults (15 years old or older) and children of 7 years old or older, there is a method of instilling 1 to 3 drops, 1 or 2 drops, or 2 or 3 drops at a time 1 to 4 times or 5 or 6 times a day and using the ophthalmic composition.

### [Container]

The ophthalmic composition according to the present embodiment is provided by being stored in a container in which a portion in contact with the ophthalmic composition is partly or entirely formed of plastic (also simply referred to as a "plastic container").

Examples of a polymer constituting the plastic include polyethylene terephthalate (PET), polybutylene terephthalate (PBT), polyethylene naphthalate, polyarylate, polycarbonate, polyethylene (PE, high density polyethylene (HDPE), low density polyethylene (LDPE), or linear low density polyethylene (LLDPE)), polypropylene (PP), polystyrene (PS), acrylonitrile butadiene styrene (ABS), polymethylpentene (PMP), polyimide (PI), a cyclic olefin polymer (COP), a cyclic olefin copolymer (COC), a copolymer of monomers constituting these polymers, and a mixture of two or more of these polymers. As the polymer constituting the plastic, polyethylene (PE), polypropylene (PP), and a cyclic olefin copolymer (COC) are preferable. In addition, the plastic may contain an elastomer.

The plastic may contain an additive such as a stabilizer. In addition, the plastic may be reinforced by containing a reinforcing agent such as glass fiber.

As the plastic, a commercially available plastic can be used without particular limitation.

The plastic container for storing the ophthalmic composition may be a container commonly used in the field of ophthalmology, and specifically, may be, for example, an eye drop container or an eye wash solution container. The type of the container is preferably an eye drop container.

In the plastic container according to the present embodiment, a portion in contact with the ophthalmic composition is partly or entirely formed of plastic. For example, in a case where the plastic container is a container having a perforated inner plug (nozzle), only a perforated inner plug portion may be formed of plastic, a storing portion or the like other than the perforated inner plug may be formed of plastic, or the container may be entirely formed of plastic.

In the plastic container according to the present embodiment, the portion in contact with the ophthalmic composition may be partly formed of plastic, but from the viewpoint of more remarkably exhibiting the effect of the present invention, it is preferable that the portion in contact with the ophthalmic composition is entirely formed of plastic. In addition, the plastic container may be formed of one type of plastic, or may be formed of two or more types of plastic.

The shape and volume of the container are not particularly limited, and may be appropriately set according to the use. In addition, the container may be a container (multi-dose container) in which a plurality of doses of the ophthalmic composition are stored, or may be a container (unit dose container) in which a single dose of the ophthalmic composition is stored.

When the container is a multi-dose container, for example, a volume may be 1.5 to 7.5 mL, 2 to 6 mL, or 2.5 to 5.0 mL. In addition, when the container is a unit dose container, a volume may be, for example, 0.1 to 1.0 mL, 0.2 to 0.9 mL, or 0.3 to 0.8 mL.

The ophthalmic composition according to the present embodiment can also be provided as a packaged ophthalmic composition. The present invention can also be regarded as an ophthalmic product (collyrium or the like) storing the ophthalmic composition of the present invention in a container.

### [2. Method for Improving Stability of Tramadol or Salt Thereof in Ophthalmic Composition]

In an embodiment, when the ophthalmic composition containing (A) tramadol or a salt thereof is stored in a container formed of plastic, the stability of the tramadol or the salt thereof in the ophthalmic composition is improved by adjusting a pH of the ophthalmic composition to 4.0 to 6.8. Therefore, as an embodiment of the present invention, there is provided a method for improving stability of (A) tramadol or a salt thereof in an ophthalmic composition, the method including setting a pH of the ophthalmic composition containing (A) the tramadol or the salt thereof stored in a container formed of plastic to 4.0 to 6.8.

The type and content of the component (A), the type and content of other components, and the preparation form and use of the ophthalmic composition in the method are as described in [1. Ophthalmic Composition].

### Examples

Hereinafter, the present invention will be specifically described based on test examples, but the present invention is not limited thereto. In addition, unless otherwise specified, a unit of each component in the table is w/v%.

### [Test Example 1: Harsh Thermal Test]

Ophthalmic compositions having the compositions shown in Tables 1 to 3 were prepared according to a conventional method. Each of the prepared ophthalmic compositions was filtered through a 0.2 µm membrane filter and sterilized. Thereafter, the ophthalmic composition was filled in a glass ampoule and an eye drop bottle (material: polyethylene, polypropylene, or COC, volume: 5 mL), and stored under the conditions described in the upper column of the table. A content of tramadol hydrochloride contained in the ophthalmic composition immediately after preparation and the ophthalmic composition after storage was quantified by HPLC, and calculated as a tramadol hydrochloride residual ratio according to the following formula. In addition, in Test Examples 4 to 10, residual improvement (%) was determined according to the following formula based on the tramadol hydrochloride residual ratio. The results are shown in Tables 1 to 3. Tramadol hydrochloride residual ratio (%) = (Tramadol hydrochloride content of storage product/Tramadol hydrochloride content immediately after preparation × 100) Residual improvement (%) in Test Examples 5 and 6 = Residual ratio in each Test Example - Residual ratio in Test Example 4 Residual improvement (%) in Test Example 8 = Residual ratio in Test Example 8 - Residual ratio in Test Example 7 Residual improvement (%) in Test Example 10 = Residual ratio in Test Example 10 - Residual ratio in Test Example 9

**[Table 1]**

| | 50°C, two months | | |
|---|---|---|---|
| | Test Example 1 | Test Example 2 | Test Example 3 |
| Tramadol hydrochloride | 0.1 | 0.1 | 0.1 |
| Boric acid | 1 | 1 | 1 |
| Borax | - | 0.005 | 0.006 |
| Sodium chloride | 0.3 | 0.3 | 0.3 |
| Hydrochloric acid | Appropriate amount | Appropriate amount | Appropriate amount |
| Sodium hydroxide | Appropriate amount | Appropriate amount | Appropriate amount |
| Purified water | Remaining amount | Remaining amount | Remaining amount |
| Total amount | 100 mL | 100 mL | 100 mL |
| pH | 4.7 | 5.9 | 7.0 |
| Container | Glass | Glass | Glas s |
| Residual ratio (%) | 99.7 | 99.9 | 100 |

**[Table 2]**

| | 50°C, two months | | | 50°C, one month | | | |
|---|---|---|---|---|---|---|---|
| | Test Example 4 | Test Example 5 | Test Example 6 | Test Example 7 | Test Example 8 | Test Example 9 | Test Example 10 |
| Tramadol hydrochloride | 0.1 | 0.1 | 0.1 | 0.3 | 0.3 | 0.3 | 0.3 |
| Sodium dihydrogen phosphate hydrate | - | - | - | - | 0.3 | - | 0.3 |
| Sodium hydrogen phosphate hydrate | - | - | - | 0.14 | 0.14 | 0.14 | 0.14 |
| Boric acid | 1 | 1 | 1 | - | - | - | - |
| Borax | 0.006 | | 0.005 | - | - | - | - |
| Sodium chloride | 0.3 | 0.3 | 0.3 | 0.65 | 0.65 | 0.65 | 0.65 |
| Potassium chloride | - | - | - | 0.1 | 0.1 | 0.1 | 0.1 |
| Chlorhexidine gluconate solution | - | - | - | 0.01 | 0.01 | 0.01 | 0.01 |
| Hydrochloric acid | Appropriate amount | Appropriate amount | Appropriate amount | Appropriate amount | Appropriate amount | Appropriate amount | Appropriate amount |
| Sodium hydroxide | Appropriate amount | Appropriate amount | Appropriate amount | Addition | Appropriate amount | Addition | Appropriate amount |
| Purified water | Remaining amount | Remaining amount | Remaining amount | Remaining amount | Remaining amount | Remaining amount | Remaining amount |
| Total amount | 100 mL | 100 mL | 100 mL | 100 mL | 100 mL | 100 mL | 100 mL |
| pH | 6.9 | 4.8 | 5.9 | 7.0 | 6.0 | 7.0 | 6.0 |
| Container | PE | PE | PE | PE | PE | COC | COC |
| Residual improvement (%) | - | 5.9 | 5.7 | - | 7.4 | - | 8.3 |

**[Table 3]**

| | 50°C, one month | |
|---|---|---|
| | Test Example 13 | Test Example 14 |
| Tramadol hydrochloride | 0.1 | 0.1 |
| Boric acid | 1 | 1 |
| Borax | 0.005 | 0.02 |
| Sodium chloride | 0.3 | 0.3 |
| Hydrochloric acid | Appropriate amount | Appropriate amount |
| Sodium hydroxide | Appropriate amount | Appropriate amount |
| Purified water | Remaining amount | Remaining amount |
| Total amount | 100 mL | 100 mL |
| pH | 6.0 | 6.5 |
| Container | PE | PE |
| Residual ratio (%) | 100 | 99.8 |

When the tramadol hydrochloride-containing ophthalmic composition was stored in a glass container, a tramadol hydrochloride residual ratio was high and stable at any pH. On the other hand, when the tramadol hydrochloride-containing ophthalmic composition was stored in a polyethylene container or a COC container, a tramadol hydrochloride residual ratio decreased at a pH of 6.9 to 7.0, but the stability of tramadol was improved by setting the pH to 6.8 or less.

### [Test Example 2: Harsh Thermal Test 2]

Ophthalmic compositions having the compositions shown in Tables 4 to 6 were prepared according to a conventional method. Each of the prepared ophthalmic compositions was filtered through a 0.2 µm membrane filter and sterilized. Thereafter, the ophthalmic composition was filled in an eye drop bottle (material: polyethylene, polypropylene, or polyethylene terephthalate, volume: 5 mL), and stored under the conditions described in the upper column of the table. A content of tramadol hydrochloride contained in the ophthalmic composition immediately after preparation and the ophthalmic composition after storage was quantified by HPLC, and calculated as a tramadol hydrochloride residual ratio according to the following formula. In addition, in Test Examples 15 to 17 and 20 to 24, residual improvement (%) was determined according to the following formula based on the tramadol hydrochloride residual ratio. The results are shown in Tables 4 to 6. Tramadol hydrochloride residual ratio (%) = (Tramadol hydrochloride content of storage product/Tramadol hydrochloride content immediately after preparation × 100) Residual improvement (%) in Test Examples 16 and 17 = Residual ratio in each of Test Examples 16 and 17 - Residual ratio in Test Example 15 Residual improvement (%) in Test Examples 21 to 23 = Residual ratio in each of Test Examples 21 to 23 - Residual ratio in Test Example 20

**[Table 4]**

| | 50°C, one month | | |
|---|---|---|---|
| | Test Example 15 | Test Example 16 | Test Example 17 |
| Tramadol hydrochloride | 1 | 1 | 1 |
| Boric acid | 1 | 1 | 1 |
| Borax | 0.008 | 0.008 | 0.008 |
| Sodium chloride | 0.2 | 0.2 | 0.2 |
| Hydrochloric acid | Appropriate amount | Appropriate amount | Appropriate amount |
| Sodium hydroxide | Appropriate amount | Appropriate amount | Appropriate amount |
| Purified water | Remaining amount | Remaining amount | Remaining amount |
| Total amount | 100 mL | 100 mL | 100 mL |
| pH | 8.0 | 4.0 | 6.1 |
| Container | PE | PE | PE |
| Residual improvement (%) | - | 22.1 | 21.9 |

**[Table 5]**

| | 50°C, one month | |
|---|---|---|
| | Test Example 18 | Test Example 19 |
| Tramadol hydrochloride | 3 | 1 |
| Boric acid | 1 | 1 |
| Borax | 0.008 | 0.008 |
| Sodium chloride | - | 0.2 |
| Hydrochloric acid | Appropriate amount | Appropriate amount |
| Sodium hydroxide | Appropriate amount | Appropriate amount |
| Purified water | Remaining amount | Remaining amount |
| Total amount | 100 mL | 100 mL |
| pH | 6.1 | 6.1 |
| Container | PE | PET |
| Residual ratio (%) | 100.1 | 100.4 |

**[Table 6]**

| | 50°C, two months | | | |
|---|---|---|---|---|
| | Test Example 20 | Test Example 21 | Test Example 22 | Test Example 23 |
| Tramadol hydrochloride | 0.05 | 0.05 | 0.05 | 0.05 |
| Boric acid | 1 | 1 | 1 | 1 |
| Borax | 0.008 | 0.008 | 0.008 | 0.008 |
| Sodium chloride | 0.3 | 0.3 | 0.3 | 0.3 |
| Hydrochloric acid | Appropriate amount | Appropriate amount | Appropriate amount | Appropriate amount |
| Sodiumhydroxide | Appropriate amount | Appropriate amount | Appropriate amount | Appropriate amount |
| Purified water | Remaining amount | Remaining amount | Remaining amount | Remaining amount |
| Total amount | 100 mL | 100 mL | 100 mL | 100 mL |
| pH | 1.0 | 4.0 | 6.1 | 8.0 |
| Container | PP | PP | PP | PP |
| Residual improvement (%) | - | 3.9 | 3.5 | -10.6 |

When the tramadol hydrochloride-containing ophthalmic composition was stored in a polyethylene container or a polypropylene container, a tramadol hydrochloride residual ratio decreased at a pH of 8.0 or 1.0, but the stability of tramadol was improved by setting the pH to 4.0 to 6.8. In addition, a tramadol analog (undetermined structure) was detected at a pH of 1.0, whereas no tramadol analog was detected at a pH of 4.0 and 6.1.

## Claims

1. An ophthalmic composition comprising tramadol or a salt thereof, wherein the ophthalmic composition has a pH of 4.0 to 6.8 and is stored in a container in which a portion in contact with the ophthalmic composition is partly or entirely formed of plastic.

2. The ophthalmic composition according to claim 1, wherein the plastic is at least one selected from the group consisting of polyethylene, polypropylene, and a cyclic olefin copolymer.

3. The ophthalmic composition according to claim 1 or 2, further comprising a buffer.

4. The ophthalmic composition according to claim 1 or 2, wherein a content of the tramadol or the salt thereof is 0.01 w/v% to 10 w/v% based on a total amount of the ophthalmic composition.

5. A method for improving stability of tramadol or a salt thereof in an ophthalmic composition, the method comprising setting a pH of the ophthalmic composition containing the tramadol or the salt thereof stored in a container formed of plastic to 4.0 to 6.8.

6. The method according to claim 5, wherein the plastic is at least one selected from the group consisting of polyethylene, polypropylene, and a cyclic olefin copolymer.

7. The method according to claim 5 or 6, wherein the ophthalmic composition further contains a buffer.
